# EUROPEAN PATENT APPLICATION

(11) **EP 4 570 173 A2**
(43) Date of publication of application: **18.06.2025**
(21) Application number: 25173828.2
(22) Date of filing: 20.03.2023
(51) Int. Cl.: A61B 5/055

(54) **WEARABLE THIN-FILM MAGNETIC RESONANCE IMAGING (MRI) RECEIVE COIL INTEGRATED WITH MRI GUIDED TRANSCRANIAL FOCUSED ULTRASOUND (TFUS ) THERAPY SYSTEM**

(30) Priority: 21.03.2022 US 202263322069 P; 10.03.2023 US 202318119833
(62) Divisional of application: 23775525.1
(71) Applicant: Mbinsight Systems, Inc., Fremont, CA 94539 (US)
(72) Inventor: CHANG, Hsu, Fremont, CA 94539 (US); HUANG, San-chao, 33845 Taoyuan (TW); YAO, Ching, 350 Miaoli (TW)
(74) Representative: Lewis Silkin LLP

(57) **Abstract**

Provided is a magnetic resonance imaging (MRI) receiver coil device and methods of manufacture. An MRI receiver coil device comprises a thin-film substrate layer configured in a dome shape and a coil array positioned around a circumference of an exterior surface of the thin-film substrate layer. A thin-film cover layer is positioned over the exterior surface of the thin-film substrate layer such that the coil array is positioned between the thin-film cover layer and the thin-film substrate layer. The MRI receiver coil device further comprises an end ring engaging the thin-film substrate layer and the thin-film cover layer such that a watertight seal is formed around the coil array between the thin-film substrate layer, the thin-film cover layer, and the end ring. The coil array comprises a plurality of coil elements, each coil element comprising a loop of conductive trace material with at least one capacitive segment.

## Description

### CROSS-REFERENCE TO RELATED APPLICATIONS

This Patent Application claims the benefit of priority to U.S. Patent Application No. 18/119,833, filed March 10, 2023, which claims the benefit of priority to U.S. Provisional Patent Application No. 63/322,069, filed March 21, 2022, the disclosure of each are hereby incorporated by reference in this Patent Application.

### TECHNICAL FIELD

This disclosure relates to magnetic resonance imaging (MRI) guided transcranial focused ultrasound (tFUS) therapy systems and methods, and more specifically to an MRI receive coil array that is tightly integrated with the tFUS therapeutic transducer device.

### BACKGROUND

High intensity focused ultrasound (HIFU) had been proposed and demonstrated as early as in 1957 to deliver focused ultrasound energy from outside of the body to a focal spot in the targeted area inside the body for thermal treatment. The temperature of the target lesion can be raised to above 60 degrees Celsius (°C) in a few seconds causing tissue to undergo necrosis. Thus, HIFU has been used as one of several thermal ablation modalities for various tumors, even though it was initially applied to brain for neurological disorders. Comparing to other traditional surgical operations, HIFU offers a minimum invasive, outpatient alternative with no incision or exposure to ionization radiation, minimum side effect and thus fast recovery. However, the applications of HIFU had not been widely accepted clinically until the use of Magnetic Resonance Imaging (MRI) to guide and monitor tissue temperature and degree of damage.

### SUMMARY

Disclosed is a Magnetic Resonance Imaging (MRI) receiver coil device for use in an MRI guided transcranial focused ultrasound (tFUS) system, and methods for manufacturing the same. The described receiver coil device is wearable, hydrophobic, and ultrasound transparent, and comprises an MRI receiver coil or MRI receiver coil array. In one embodiment, an MRI receiver coil device includes a first inner layer of flexible substrate made of a hydrophobic and ultrasound transparent thin film and fabricated to conform to fit to a certain 3D shape, such as that of the head of a subject or individual patient. A pattern of conductive material may be formed on a first surface of the first inner layer. The pattern may include at least one receiver coil and at least one capacitor, and the conductive traces so formed may comprise multiple conducting layers and at least a layer of dielectric plastic material. A second thin-film outer layer of the same material of the first inner layer is formed over the conductive pattern. The three-dimensional (3D) geometric configuration of the receiver coil device may conform to the 3D geometric configuration of a head model rendered from a 3D image data set of the individual patient. In an alternate embodiment, the thin-film coil device is fabricated upon a fixed 3D shape, and a gel-like ultrasound transparent material is used to fabricate an individually shaped thin layer of gel pad lining that is conformed to individual patient's head on the inside and to the fixed-shape thin-film coil device on the outside. The described methods may utilize 3- to up to 6-dimensional printing technique or other precision multi-dimensional fabrication techniques.

### BRIEF DESCRIPTION OF DRAWINGS

Various embodiments are described and illustrated herein with reference to the drawing in which like items are indicated by the same reference numeral, and in which:
FIGS. 1A and 1B illustrate an example transcranial focused ultrasound (tFUS) device, in accordance to one or more embodiments.
FIGS. 2A and 2B illustrate example thin-film coil array assemblies, with associated tFUS transducers, in accordance with one or more embodiments.
FIG. 3 illustrates an example process for constructing a customized thin-film coil array assembly, in accordance with one or more embodiments.
FIG. 4 illustrates an example 3D MRI image dataset and resulting surface rendering, in accordance with one or more embodiments.
FIGS. 5A, 5B, 5C, and 5D illustrate an example 3D printed head mold and thin-film layers fabricated thereon, in accordance with one or more embodiments.
FIG. 6 illustrates a cross-sectional layered view of successive layers of a single coil element of an example coil array, in accordance with one or more embodiments.
FIG. 7 illustrates a top view of an example coil array configuration with adjacent overlapping coil loop elements, in accordance with one or more embodiments.
FIG. 8A illustrates an enclosed end ring for holding tune/match circuit boards, in accordance with one or more embodiments.
FIG. 8B illustrates a typical tune/match circuit board for one channel of an example receive-only coil array element, in accordance with one or more embodiments.
FIG. 8C shows an example thin-film coil array assembly with an end ring fixed on a coil array cap, in accordance with one or more embodiments.
FIG. 8D illustrates a cross-sectional view of an interface configuration between an end ring and a coil array cap, in accordance with one or more embodiments.
FIG. 9 illustrates an interface between a customized thin-film coil array assembly and the frame of a tFUS transducer device, in accordance with one or more embodiments.
FIG. 10 illustrates an example process for constructing a universal thin-film coil array assembly, in accordance with one or more embodiments.
FIGS. 11A and 11B illustrate an example universal thin-film coil array constructed over a fixed shape mold, in accordance with one or more embodiments.
FIG. 12 illustrates an example concave mold for forming a wearable customized liner, in accordance with one or more embodiments.
FIG. 13 illustrates an example flow process for placement of a universal thin-film coil array assembly onto a patient's head, in accordance with one or more embodiments.
FIG. 14 illustrates an interface between a universal thin-film coil array assembly and the frame of a tFUS transducer device, in accordance with one or more embodiments.

### DETAILED DESCRIPTION

In the following description, numerous specific details are set forth in order to provide a thorough understanding of the present disclosure. The present disclosure may be practiced without some or all these specific details. For the purpose of clearly describing the present disclosure, well known process operations generally practiced in the art are not described in detail so as to not unnecessarily obscure the described concepts. While some concepts will be described in conjunction with the specific embodiments, it will be understood that these embodiments are not intended to be limiting. On the contrary, it is intended to cover alternatives, modifications, and equivalents as may be included within the spirit and scope of the present disclosure as defined by the appended claims.

For example, the techniques of the present disclosure will be described in the context of focused ultrasound therapy on various body parts, such as the head and brain. However, it should be noted that the techniques and mechanisms of the present disclosure apply to various other body parts, such as the abdomen or torso. Various techniques and mechanisms of the present disclosure will sometimes be described in singular form for clarity. However, it should be noted that some embodiments include multiple iterations of a technique or multiple instantiations of a mechanism unless noted otherwise.

### Overview

The advantage of magnetic resonance imaging (MRI) to guide thermal therapy goes beyond its ability to provide fast and accurate temperature imaging of the treatment target inside the patient's body during ablation procedures. As a proven imaging modality with the best soft tissue contrast, MRI scans are used to plan the treatment, to assess the treatment outcome immediately after, and for long-term follow-up. Moreover, MRI can provide various additional contrast mechanisms such as those for protein denaturation and lesion formation to enable more control over the treatment outcome even during the ablation procedure. Another MRI approach, magnetic resonance acoustic radiation force imaging (MR-ARFI), can detect small focal displacements induced by low-power ultrasound pulses. Comprehensive MRI thermometry, in conjunction with other acoustic measuring techniques, has been proven to be the ideal imaging modality to guide and navigate various focused ultrasound (FUS) therapeutic procedures, including but not limited to high intensity focused ultrasound (HIFU) ablation procedures.

Several MR-guided HIFU devices have been developed, mainly for abdominal target areas. Existing methods have utilized a general-purpose MRI-compatible robotics system to move and operate the therapeutic or diagnostic devices inside the MRI bore. Other methods have utilized a three-axes motion stage to position the HIFU transducer in an unsealed water tank, which can be put onto the MRI patient couch. An arc structure may hold the HIFU transducer and to allow mechanical movement control up to 6 degrees of freedom while being seated on the patient couch inside the bore of existing MRI systems. An MRI receive-only phased array coil may be used to improve MRI signal-to-noise ratio (SNR). A smaller coil gets higher SNR with smaller sensitivity volume, but, comparing the SNR over same region of interest, combining an array of multiple small coils exhibits better SNR than a big coil alone. Another advantage of a multiple-channel coil array is that it can be used for parallel imaging to accelerate the MRI scan. For example, a 32-channel head array coil arranged in a soccer-ball pattern with overlapping loops may demonstrate higher SNR and capability of high acceleration factor in parallel imaging.

For abdominal HIFU applications under MRI guidance, it is essential for the MRI receiving coil configuration to integrate with the HIFU transducer device which is closely positioned on the treated anatomy and can be moved during the treatment to focus on sequential sonication points in a target region of interest. An N+1 multiple-channel configuration may be used to take advantage of a hollow loop coil that moves with the transducer HIFU device while allowing the ultrasound beams to go through un-impeded as well as serving as additional parallel imaging channel to a multiple-channel (N) coil array that stays fixed in position.

However, the aforementioned systems are not designed for application within the brain. In recent years, focused ultrasound, also under MRI navigation, has been applied to treat various neurological and psychiatric disorders in a transcranial focused ultrasound (tFUS) transducer array configuration. Such a tFUS transducer device is placed on top of the patient's head, and equipped with multiple transducer elements transmitting multiple ultrasound beams through a water bath to penetrate the skull. In this transcranial configuration, tight integration of MRI head coil array with tFUS transducer device is critical for the demanding treatment precision in order to take full advantage of MRI capability for image navigation, including: targeting, positioning, aiming, real-time monitoring and dose control, immediate prognosis, and follow up. Not only does advanced MRI technology play a pivotal role in targeting defective neural circuitry to be treated in support of the soft tissue contrast provided by usual anatomic structural images, but thermometry and/or acoustic dosage monitoring have also become increasingly demanding in speed and sensitivity.

MRI guided tFUS was approved by FDA for the treatment of essential tremor in 2016, and Parkinson's disease with main symptom of tremor in 2018. However, during treatment, MRI scans can only be performed with either the large size whole body MRI coil or a simple two-loop MRI coil inserted in the water bath of the tFUS transducer device. A typical MRI multiple-channel head coil is itself a helmet like structure and cannot be placed simultaneously with the tFUS transducer helmet. Thus, MRI images may be obtained prior to ultrasound treatment. However, where the images used for targeting or treatment planning are acquired with head coil prior to treatment, the actual position of the head during treatment when tFUS transducer is in place could exhibit a mis-registration on the images used for planning. A stereotactic frame is required and anchored onto patient skull to ensure the precision of the treatment target locations. Additionally, once the tFUS is in place, MRI sensitivity can no longer be fully realized with body coil or simple two-loop coil let alone full signal-to-noise ratio (SNR) and acceleration performance.

Some methods have proposed using a soft 8-channel coil wrapped around the lower part of the head and face of the patient and positioned external to the water bath of the tFUS transducer placed over the top of the head, in an effort to minimize direct interference between the magnetic field of view of the coil and the acoustic field of coverage from the hemispherical tFUS transducer. Because the coil is placed externally to the tFUS transducer, this configuration prevents complete imaging of certain substantive portions of the brain that may be the target of treatment. Additionally, even though the thin wire coil exhibited high acoustic transparency, the fabric like material used to embed thin wire coil elements and wrap around the patient's face is air permeable and therefore not hydrophobic or ultrasound transparent. Thus, the coil cannot be immersed in the water bath within the tFUS transducer, or otherwise placed within the tFUS transducer, to improve imaging of the treatment target.

However, with proper choice of thin enough layers of conducting and dielectric material for coil fabrication, a coil can be placed within the tFUS transducer and can yield low attenuation of the ultrasound beams through the coil traces for tFUS transducer. The present disclosure provides a configuration for a thin-film coil array that is positioned within a tFUS transducer device that provides improved signal-to-noise-ratio (SNR) performance and allows for parallel imaging acceleration to enable a high temporal resolution for the navigation MRI scans. The described systems and methods achieve a tight integration of the MRI coil array into the tFUS transducer device, while preserving or satisfying the following requirements:
- array of 8 to 16 or more MRI signal receiving channels
- positioned in close proximity to the head
- fixed in position with rigidity despite water bath to ensure RF receiving stability
- minimized acoustic attenuation to ensure acoustic beam penetration and adequate focusing
- minimized interference from transducer

In a nutshell, the described systems and devices may include or implement the following elements:
- a solid 3D model constructed from an individual patient's 3D head MRI images for providing the exterior surface shape of the head;
- a thin-film layer fabricated on top of the head mold with a material that is both hydrophobic and acoustic transparent, including but not limited to TPU (Thermoplastic Polyurethane), various ultrasound gel material, and so on; where the fabricating technique employed in this and subsequent steps is 3D, or up to 6D, printing technique;
- a first layer of conducting material fabricated on top of the thin film to form the base trace of coil pattern, followed by the layer of dielectric segments for capacitors, and then, the overlapping layer of the conducting material;
- a second layer of thin film fabricated upon the first thin-film layer and conducting material forming a thin-film coil array with elastic, hydrophobic and ultrasound transparent characteristics much like a soft cap;
- an end ring, a solid structure that holds electric circuits for the coil elements, that is attached to seal the conducting material within the thin-film coil array cap;

The fabrication technique used for this thin-film layers and conducting material is not the silk-screen planar printing typically used for flexible printed circuit board on a planar substrate surface. Instead, it employs high-precision multi-dimensional (3D up to 6D) printing technology. The thin-film coil array assembly may then be placed onto patient's head before sealing onto the frame of the tFUS device.

In an alternate embodiment, a convex mold is constructed based on a defined 3D shape, on which to fabricate a universal thin-film coil array, and its concave counterpart is used with the head mold to form a void space in which to fill with an ultrasound transparent gel-like material to fabricate a customized gel pad liner. As an alternative embodiment, a gel-material layer of liner can be fabricated on top of the head mold to conform externally to the defined 3D shape of the universal coil array.

### Example Embodiments

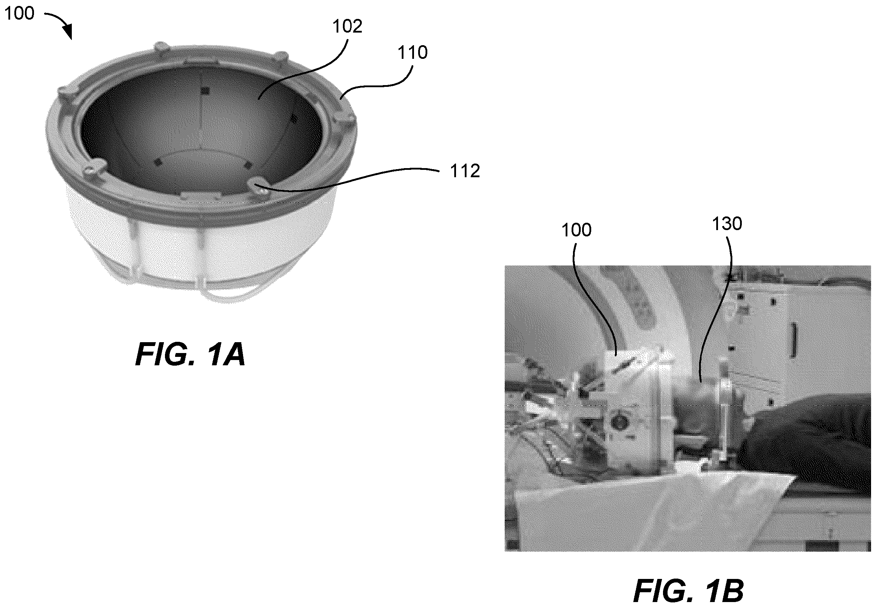

FIGS. 1A and 1B illustrate an example transcranial FUS (tFUS) device 100, in accordance with one or more embodiments. FIG. 1A shows a perspective view of tFUS device 100 showing a cavity 102 in which a subject's head is placed. The tFUS device 100 further includes a frame 110 with attachment mechanism 112 for interfacing with another frame that holds a subject-interfacing membrane. FIG. 1B shows a side view of tFUS device 100 positioned on the head of a subject 130. As described herein, a subject of the described systems and devices may be referred to as a "patient," or a "user." In order to take full advantage of image navigation capability of MRI for advanced transcranial focused ultrasound (tFUS) therapy of various neuropsychiatric disorders, especially for real-time monitoring of tissue temperature, degree of necrosis, or effect of ultrasonic neural modulation during treatment, optimal performance in receiving signal-to-noise-ratio (SNR) become increasingly essential for the required spatial and temporal resolution. This is typically achieved by equipping the clinical MRI system with a head specific radiofrequency (RF) receiving coil array. A specific coil array may include multiple elements or channels, up to 32 channels or more, for head and/or brain scans. However, with the presence of tFUS transducer device, tight integration has been challenging in view of the complexity of mutual compatibility of the two modalities. The described coil array assemblies resolve such issues with respect to the optimal location, stable support, water proof, and ultrasound transparency of a multiple channel coil array with minimum change to existing tFUS transducer configurations.

Since coils need to be placed inside of the transducer device and close to the head, it has become quite a challenge to address the issue associated with the coil array being positioned in the path of ultrasound beams emitted from the transducer device and thus often inevitably submersed in the water bath, which is used as an ideal interface media to patient head for ultrasound beam emitted from the transducer elements.

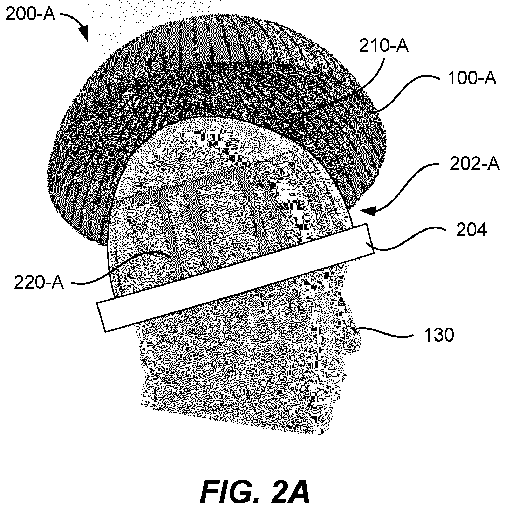

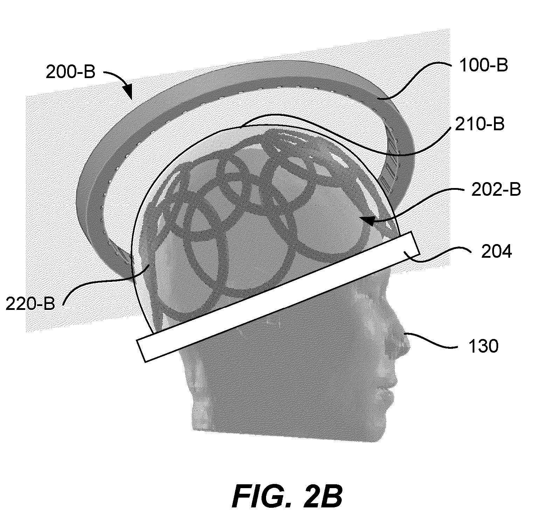

With reference to FIGS. 2A and 2B, shown are example thin-film coil array assemblies 200-A and 200-B, in accordance with one or more embodiments. FIG. 2A illustrates a wearable thin-film coil array assembly 200-A having a coil array 220-A arranged in a cylindrical configuration. Assembly 200-A may be integrated with a conventional tFUS transducer array 100-A distributed on a hemi-spherical surface. As used herein, a thin-film coil array assembly may be referred to as "coil array assembly" or "MRI receiver coil device." As shown in FIG. 2A, coil array assembly 200-A is positioned, or worn, on the head of subject 130. In various embodiments, coil array assembly 200-A comprises thin-film cap 202-A coupled to end ring 204. In an embodiment, thin-film cap 202-A comprises coil array 220-A of one or more coil elements (including conductive loops and serial capacitors) embedded in between two thin-film layers 210-A. In some embodiments, the thin-film layers are fabricated from a selected material, such as a thermoplastic. For example, thermoplastic polyurethane (TPU), which is known to exhibit high hydrophobicity as well as minimum ultrasound attenuation in a thin film form, may be used. Furthermore, the described systems and methods are not limited to the use of TPU and other selected materials, including polymers and fluoropolymers, may be chosen based on various properties, including elasticity, hydrophobicity, ultrasound transparency, and good dielectric properties. For example, the thin-film material may include, without limitation, one or more of the following: polyimide (PI) film, a polyethylene (PE) film, a polyethylene terephthalate (PET) film, a polyethylene naphthalate (PEN) film, a polyetherimide (PEI) film, a polyphenylene sulfide (PPS) film, a polytetrafluoroethylene (PTFE) film, and a polyether ether ketone (PEEK) film. In some embodiments, the conducting loops and serial capacitors are fabricated between the two layers of the thin film as further described below.

In example embodiments, the coil array assembly includes a minimum of 8 channels. The number of channels is not limited to 8 and the described coil array assemblies may have fewer channels, or more channels, than 8 (such as 4 or 16 channels depending on specific applications). As shown in FIG. 2A, the element loops of the coil array may be arranged in a cylindrical configuration to be positioned around the side of a patient's head. This cylindrical configuration may maintain as much orthogonality as possible between the radiofrequency (RF) magnetic field direction and the direction of the ultrasound beams when tFUS transducer 100-A, itself also an array of transducer elements, is distributed in a hemi-spherical configuration covering the top of the head. Transducer 100-A may be enclosed inside the tFUS transducer device 100.

FIG. 2B shows another embodiment of a coil array assembly 200-B with coil array 220-B comprising element loops placed in an overlapping configuration within two layers of thin-film 210-B of the thin-film cap 202-B. Coil array assembly 200-B also includes end ring 204 when fully assembled. The coil array assembly 200-B has a hemi-spherical configuration that may be optimized for MR imaging of human cortical structure and implemented to function with a tFUS transducer array with elements distributed in a ring shape or cylindrical structure such as tFUS transducer 100-B. The pairings of the dual modality arrays in mutually orthogonal relative configurations, in FIGS. 2A and 2B, is a way to minimize the interference between the respective fields each produces. Cylindrical ultrasound transducer array also can produce finer and sharper acoustic focus profile for certain applications.

However, as technology moves into advanced MRI functional and structural studies of the cortical regions of the brain near the top, a coil array with 32 or more channels having a soccer-ball like configuration may demonstrate enhancement in sensitivity or acceleration. In this case, some of the element coil loops are exactly on the path of the ultrasound beams produced by the central transducer elements and minimizing ultrasound attenuation is one critical issue to resolve to integrate the two modalities. Notwithstanding careful selection of thin-film material and maintaining thinnest possible thickness for conductive traces and dielectric segments, as an alternate embodiment, disclosed herein is a ring-shaped tFUS transducer array with elements distributed in a cylindrical symmetry, which is shown as 200-B in FIG. 2B, to pair with coil array with a hemi-spherical distribution.

The described coil array assemblies also address another critical issue to minimize eddy current induced by the coil loops on the transducer hemisphere where a conducting electrode surface may exist. In reducing the eddy current effects, the electrode plate of the tFUS transducer's common ground is cut or grooved into a number of thin radial strips on the inside of the hemispherical transducer array, shown as 100-A in FIG. 2A. The radial strip-shape grooves, while keeping the ground of all the tFUS transducer elements to be connected, restrict the eddy current loop in a narrow area with minimal effect. In the case of a ring-shaped tFUS transducer, the ground is grooved into a comb shape (100-B in FIG. 2B) to achieve the same effect.

To ensure no or minimum ultrasound impeding, deflection, or attenuation, the thin-film coil array assembly, such as 200-A shown, is fabricated to be "worn" onto individual subject's head with tight fit and close contact. In some embodiments, the described coil array assembly, such as 200-B, is placed over a customized gel pad worn over the subject's head or with a close-fit gel-pad liner attached to the inside of the thin-film cap. A customized liner may allow for a close fit to the subject's head while maintaining ultrasound transparency. Furthermore, the coil array assembly must be placed inside of a tFUS transducer device and submersed within the water bath used as the interface media between the subject's head and ultrasound beams emitted by the transducer. Thus, the coil array assembly requires hydrophobic and waterproof properties to protect the coil elements from the water or other fluids. The present disclosure describes the configuration, and method of manufacturing, of a coil array assembly that is positioned close to the patient's head to satisfy the proximity principle in achieving the optimal SNR on one hand and, on the other, to meet requirements of hydrophobicity and ultrasound transparency

### Customized Thin-Film Coil Array Assembly

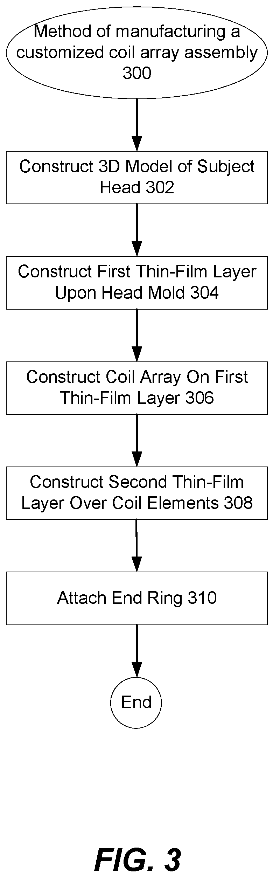

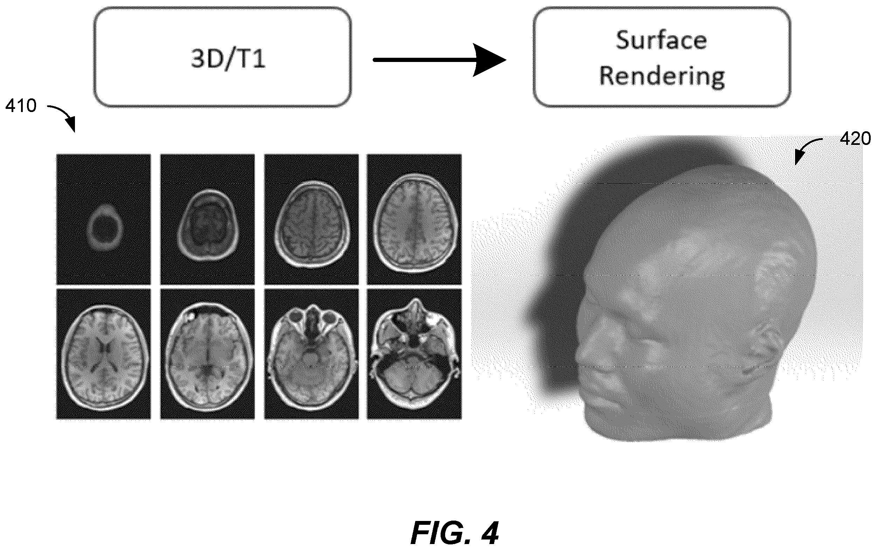

In some embodiments, the coil array assembly is customized to fit a particular subject's head. This ensures the closest overall positioning of the coil elements around the target area (i.e., subject's head). With reference to FIG. 3, shown is an example process for constructing a customized coil array assembly, in accordance with one or more embodiments. FIG. 3 is described with reference to the following FIGS. 4, 5A, 5B, 5C, 5D, 6, 7, 8A, 8B, 8C, 8D, and 9, which illustrate the configuration of the described coil array assemblies, and components, at various stages of construction.

In some embodiments, a 3D model of the subject's head is obtained to construct such a customized coil array assembly. For example, at operation 302, a 3D model of the subject's head is constructed. The 3D model is customized to a particular head and corresponding geometric configuration of a subject (or patient). Various techniques are implemented to generate the 3D model.

In one example, a 3D model is rendered using MRI image data. With reference to FIG. 4, shown is an example 3D MRI image dataset 410 and resulting surface rendering 420, in accordance with one or more embodiments. Although image dataset 410 shows eight (8) images of an axial view of the entire head of a user, such as subject 130, many more additional images are included in dataset 410. For example, a 3D MRI image dataset 410 include a total of 148 images, each corresponding to a 1.2-millimeter-thick slice. With these images, one can use a binary threshold to define the MRI null signal areas, which includes the air chambers of head areas and the head-outside areas. Then, the null signal area is processed with a "Connected Components Labeling" algorithm. By selecting the biggest area, one can get the head-outside-only images. Collecting all the head-outside-only images from top to bottom, the surface rendering of the patient's head is obtained by a "marching cube" algorithm. The marching cube algorithm calculates hundreds of thousands of triangles on the head surface and provides the 3D coordination data of those triangles to generate surface rendering 420. It should be recognized that various other techniques may be implemented to obtain the 3D model rendering of the subject's head, including 3D optical scanning.

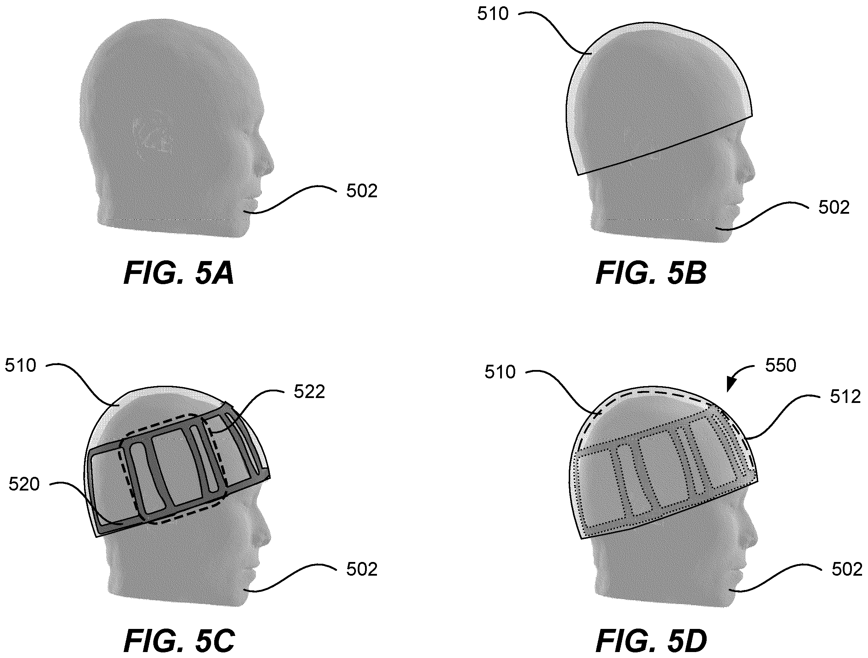

The 3D surface rendering 420 is then used to generate a physical model which functions as a mold for the coil array assembly to be built upon. FIGS. 5A, 5B, 5C, and 5D show an example 3D solid head mold 502 and thin-film layers fabricated thereon, in accordance with one or more embodiments. FIG. 5A shows 3D head mold 502, which is constructed from the 3D coordination data using a 3D printer. In various embodiments, head mold 502 is constructed from using 3D fabrication techniques, such as, but not limited to, stereolithography (SLA), selective laser sintering (SLS), fused deposition modeling (FDM), digital light process (DLP), multi jet fusion (MJF), PolyJet, direct metal laser sintering (DMLS), and electron beam melting (EBM). The head mold may be constructed from various materials suitable for the manufacturing techniques described above, such as Polylactic Acid (PLA) for example. In some embodiments, the entire head mold is not constructed. Instead, only the relevant portions of the head mold that are required for shaping and constructing the coil array assembly are constructed. Various printing techniques are implemented including stereolithography.

At operation 304, a first thin-film layer is constructed upon the head mold. FIG. 5B shows a first thin-film layer 510 of the coil array assembly that is constructed over the head mold. The first thin-film layer is referred to as a substrate layer or inner layer. As previously described, first thin-film layer 510 comprises a material that is both hydrophobic and ultrasound transparent, such as TPU. In some embodiments, the first substrate layer is fabricated via various techniques including vacuum forming, and printing with higher dimensional precision, evenly over the head mold. In other embodiments, the head mold is coated in a layer of the substrate material by dipping the head mold into a bath of the material in liquid form and then dried. In some embodiments, the first thin-film layer is coated onto the head mold by spraying the material over the desired portions of the head mold and then dried. Additional machining may be used to form or shape the material that is dipped or sprayed onto the mold. As such, the first thin-film layer will conform closely to the geometrical profile of the head mold, and thus the head of the subject.

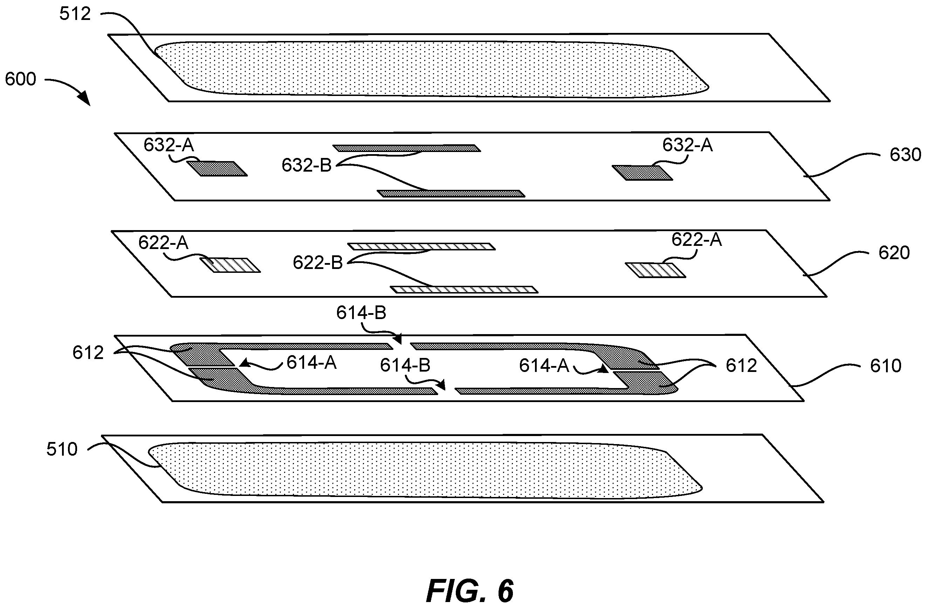

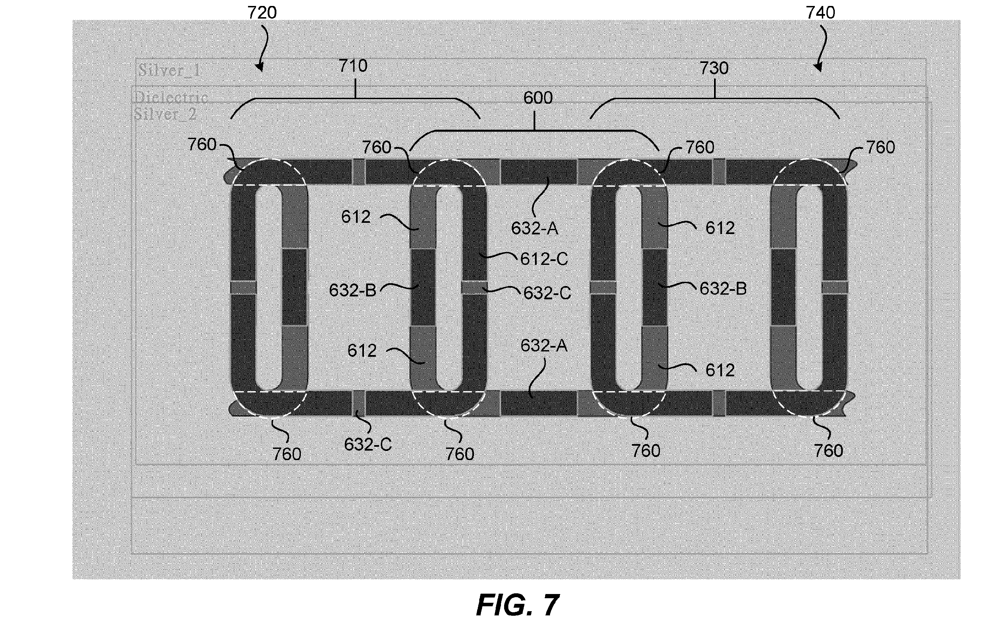

Then, at operation 306, a coil array is constructed on the first thin-film layer. FIG. 5C shows coil array 520 constructed over first thin-film layer 510. In some embodiments, the coil array is fabricated on first thin-film layer 510 using conductive ink and selected dielectric materials. Coil array 520 may comprise one or more conducting material layers for coil trace and one or more dielectric layers for capacitors. In various embodiments, the coil array comprises a pattern of conductive traces and distributed capacitors configured into one or more coil elements. The coil elements may comprise loops or other configurations of conductive traces. As shown in FIG. 5C, coil array 520 comprises multiple overlapping conductive loops, each a coil element. In an example embodiment, the coil array comprises eight overlapping coil elements evenly positioned in a cylindrical pattern surrounding the subject's head, as shown in FIG. 5C. One coil element 522 of the coil array is outlined in dashed lines in FIG. 5C. Configurations of the coil pattern are further described with reference to FIGS. 6 and 7.

With reference to FIG. 6, shown is a cross-sectional layered view of successive layers of a single coil element 600 of an example coil array, in accordance with one or more embodiments. In various embodiments, coil element 600 is an example of coil element 522 in FIG. 5C. As visualized in FIG. 6, coil element 600 is fabricated by at least three layers 610, 620, and 630, in which a middle layer of dielectric material 620 is sandwiched in between two layers of conductive material 610 and 630. Various conductive material and dielectric material may be used. For example, the conductive material may comprise, without limitation, silver, copper, graphene, and so on. For example, the dielectric material may comprise, without limitation, polytetrafluoroethylene (PTFE), or other ceramic, glass, mica, and plastic material.

These three layers of coil element 600 (traces of conductors and distributed serial capacitors) are sandwiched between two layers of thin film 510 and 512. In various embodiments, the conductive layer 610 comprises the base conductive trace of a loop with one or more gaps at selected locations along the loop. Conductive layer 610 is fabricated onto the first or inner thin-film layer. The dielectric layer 620 is fabricated on top of the conductive layer 610. The dielectric layer 620 comprises one or more dielectric segments overlapping the position of the gap or gaps in layer 610. Subsequently, a second conductive layer 630, comprising conductive segments matching the dielectric segments, is fabricated on top of the dielectric layer 620.

For the purpose of minimizing the attenuation of through-film ultrasound, the thickness of single layer in an example embodiment may be no thicker than 0.02 millimeters (mm) for conductive material (layers 610 and 630), no thicker than 0.1mm for dielectric material (layer 620), and no thicker than 0.1mm for thin-film layer material (layers 510 and 512). However, it should be recognized that the thicknesses of each of the layers and components are not limited in the described ranges and may be varied to achieve various conductive or wave transparency characteristics, as well as various structural or mechanical characteristics. For example, the dielectric material may be provided at a thickness of greater than 0.1mm.

The described systems may employ multi-dimensional printing techniques to fabricate both thin-film layers and multiple layers of the coil array onto the thin-film layers. As such, the layers are constructed to conform to the 3D shape of the head mold. Unlike the solid head mold 502 which may be constructed by conventional 3D printing techniques, each thin-film layer (510 and 512) and the coil array traces (610, 620, 630) are constructed using multi-dimensional printers, such as 5-dimensional or up to 6-dimensional printers, which are capable of performing three orthogonal translational dimensional motion in conjunction with two to three-dimensional rotational motion, on a physical 3D mold. This can be achieved by modifying a conventional 3D printer with additional fixtures for rotational control or utilizing a 6-axis robotic arm. This ensures that the thickness of a layer, such as the thin-film substrate layer, is an even thickness throughout the layer over the 3D contour of the mold.

The described fabrication techniques are distinctly different from two-dimensional (2D) planar screen-printing techniques and other planar fabrication techniques commonly used to print traces onto a thin flat substrate sheet (i.e., flexible printed circuit boards), which were then folded into non-planar shapes after printing. Because of the flexible nature of such printed circuit boards, there is a lack of mechanical stability, especially when submerged in a water bath and subjected to water pressure. Such printed circuit boards are also unable to conform accurately to a subject's head shape.

As shown in FIG. 6, coil element 600 comprises a loop configuration formed by four curved trace structures 612 in layer 610 on which one or more gaps 614-A and 614-B are designed in between the curved trace structures to position distributed serial capacitive elements, so as for the coil to resonate electrically at the Larmor frequency of the MRI scanner. To form these serial capacitive elements, a segment of overlapping conductor trace (in layer 630) is aligned with each gap to form, at the location of the gap, the equivalent of two capacitors in series with the inductive loop. As shown, overlap traces 632-A (in layer 630) are aligned with gaps 614-A with dielectric segments 622-A (in layer 620) positioned in between the overlap traces in layer 630 and the curved trace structures in layer 610. As also shown, overlap traces 632-B (in layer 630) are aligned with gaps 614-B with dielectric segments 622-B (in layer 620) positioned in between the overlap traces in layer 630 and the curved trace structures in layer 610. The capacitance of the distributed capacitive element required for resonance can be adjusted empirically with the selection and thickness of the dielectric material as well as the area of overlapping segments of the overlap traces.

In some embodiments, the dimensions of curved trace structures 612 are identical. In some embodiments, the dimensions of gaps 614-A and 614-B are the same. In some embodiments, dielectric segments 622-A and 622-B are the same. In some embodiments, the dimensions of overlap traces 632-A and 632-B are the same.

In an array of multiple coil loop elements, adjacent loops are often overlapped slightly to eliminate mutual inductance, which can interfere with the resonance. FIG. 7 shows a top view of an example coil array configuration with adjacent overlapping coil loop elements, in accordance with one or more embodiments. Coil element 600 is shown in FIG. 7 with adjacent coil elements 710 and 730 overlapping coil element 600. Components located on layer 610 are shown in gray while component located on layer 630 are shown in blue. As such, curved structures 612 of coil element 600 are shown in gray while overlap traces 632-A and 632-B are shown in blue. In one implementation, an opposite pattern is printed for adjacent coil elements in order to form the overlapping configuration. For example, the curved structures (612-C) of coil elements 710 and 730 are located within layer 630 and shown in blue, while overlap traces (632-C) of coil elements 710 and 730 are located within layer 610 and shown in gray. The pattern on the dielectric layer 620 prints dielectric traces at all capacitive segments described in FIG. 6. Dielectric traces in layer 620 are also fabricated at overlapping segments 760 wherever conductive traces of adjacent coil elements overlap (outlined in dashed lines). The described configuration minimizes the number of layers to three to achieve coil resonance without the interfering mutual inductive coupling. Minimizing the number of layers also reduces the amount of material used, as well as allows the structure of the coil array assembly to be as thin as possible in order to minimize ultrasound attenuation.

Once the coil pattern is constructed upon the first thin-film layer, a second thin-film layer is constructed over the coil elements at operation 308 for additional dielectric isolation and waterproofing. FIG. 5D shows a second thin-film layer 512 positioned over first thin-film layer 510 (shown in dashed lines) and the coil elements to seal the coil elements within the first thin-film layer and the second thin-film layer, forming the thin-film coil array cap 550. As used herein, the second thin-film layer is referred to as cover layer or outer layer. The second thin-film layer may be fabricated using any of the described construction techniques for fabricating the first thin-film layer. In some embodiments, the techniques used for fabricating the first and second thin-film layers may be the same or different.

The second thin-film layer is fabricated directly upon the first thin-film layer and coil elements. In some embodiments, air pockets or gaps between the thin-film layers and the coil elements are eliminated or reduced to the greatest extent possible. While the second thin-film layer may comprise the same material as the first thin-film layer, in some embodiments, different materials may be utilized for each thin-film layer. Although the previously discussed operations construct the thin-film cap layer by layer upon a physical head mold, in some embodiments, a physical 3D head mold may not be required. Instead, the thin-film layers and the coil array may be constructed based on the merged 3D head rendering data with 3D CAD (Computer-aided design) model.

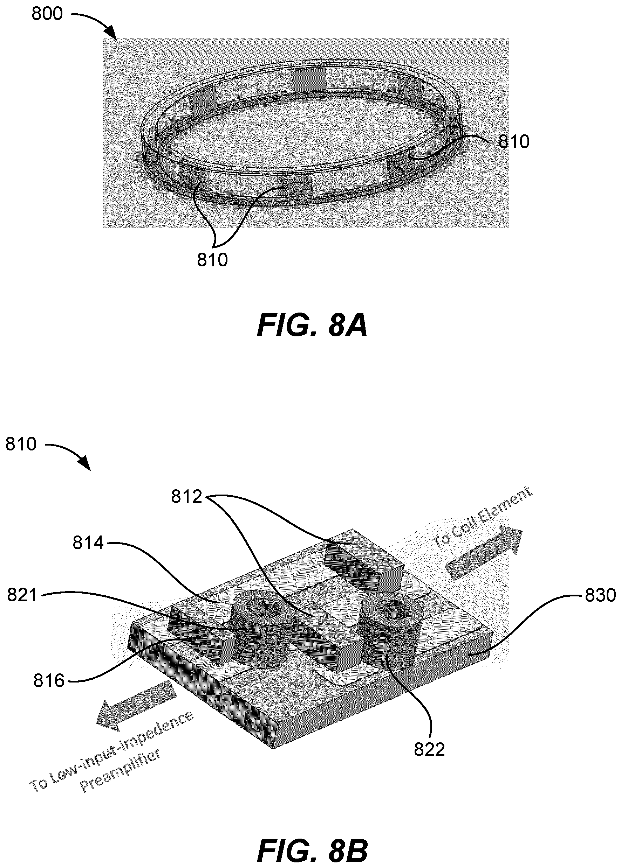

At operation 310, an end ring is attached to the thin-film layers of thin-film coil array cap 550. In this way, the coil elements are completely sealed within the two thin-film layers and the end ring. Additionally, each coil element of the coil array is electrically connected, or otherwise coupled, to one channel of the signal receiving unit of the MRI system through a tune and match circuit board (TMCB) enclosed or housed in the end ring. FIG. 8A shows an example end ring 800 that may be implemented with one or more embodiments of the present disclosure. End ring 800 includes multiple TMCBs 810 positioned along the interior circumference of the end ring. As illustrated, the structure of end ring 800 is transparent to show eight (8) TMCBs 810, each corresponding to a coil element of the coil array 520. The TMCBs are housed in the structure of end ring 800, which is a rigid hollow ring-shape structure. Each TMCB is positioned with equal, or approximately equal spacing within the structure of the end ring.

FIG. 8B shows an example tune and match circuit board (TMCB) 810, in accordance with one or more embodiments. The TMCB shown in FIG. 8B includes input connecting to one coil element and output to a preamplifier with low input impedance. The circuit board comprises ceramic capacitors 812, copper strips 814, variable capacitors 821 and 822, PIN diode 816, and printed circuit board 830. Example circuit boards include flex circuit boards and FR4 circuit boards. The TMCB makes a parallel resonance with the inductive coil element loop, yielding a high impedance for the coil element and thus a reduction of current in the coil element to eliminate the mutual inductance effect between non-adjacent coil elements. For adjacent coil elements, the overlapping method is used to eliminate the mutual inductance effect. The PIN diode 816 is used to perform active decoupling for protecting the receive-only coil element from high RF power transmitted by the transmit coil of the MRI system during transmit. On the printed circuit board 830, the electric circuit resonance can be tuned to the Larmor frequency of the MRI magnetic field by adjusting the capacitance of the variable capacitors 821 and 822, to achieve optimal pick-up of the magnetic resonance signal.

The magnetic resonance signal picked up by the coil elements is amplified by multi-stage amplifiers, including low-input-impedance preamplifier, middle amplifier, and various gain amplifiers. Then, the signal from each coil element is carried to a channel of a receiver where the magnetic resonance signal is processed by analog-to-digital sampling and digital down converter before being re-ordered to form the time-domain data. Finally, the time-domain data is Fourier transformed to reconstruct an MR imaging. The above MRI signal processing chain can be independent for each channel.

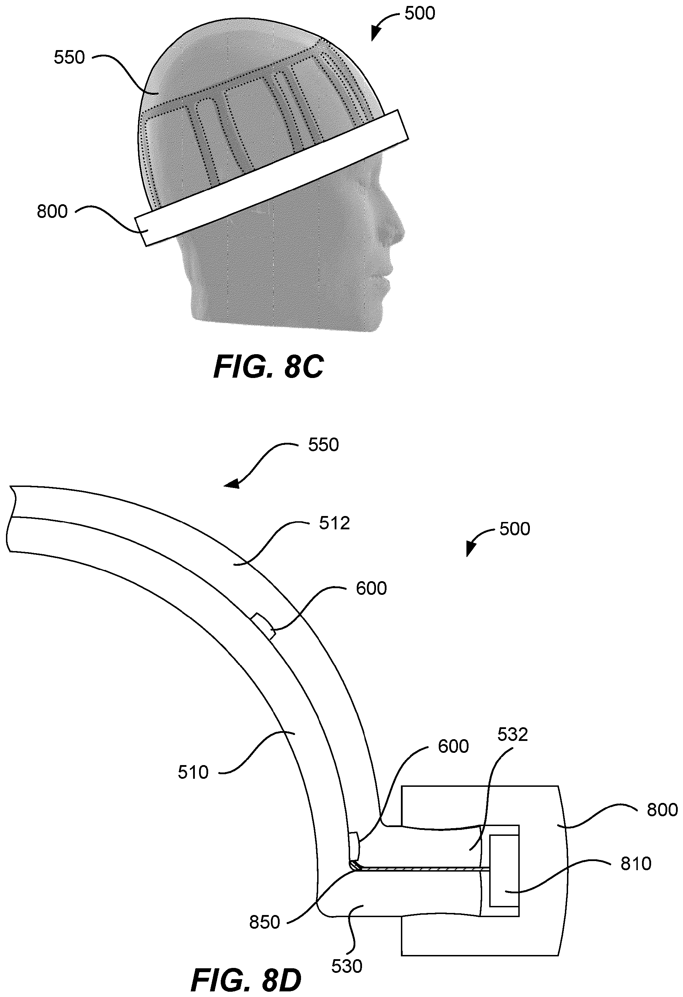

The end ring is coupled to the thin-film cap at a lip or edge structure of each thin-film layer. FIG. 8C shows end ring 800 attached to the thin-film coil cap 550 forming coil array assembly 500 customized to a particular subject's head. FIG. 8D illustrates a cross-sectional side view of the coil array assembly, in accordance with one or more embodiments. FIG. 8D shows the interface between end ring 800 and thin-film coil cap 550. In various embodiments, end ring 800 may attach to thin-film coil cap 550 at edge structures 530 and 532 extended from thin-film layers 510 and 512, respectively. The edge structures may extend around the entirety of the thin-film cap, forming a brim-like structure. In some embodiments, the edge structures may be constructed along with the thin-film layers, such as during operations 304 and 306. In some embodiments, the edge structures may be extensions or portions of respective thin-film layers.

Each TMCB in the end ring may be electrically coupled to a coil element through an additional conducting trace 850 embedded within the thin-film layers extending from the tightly fitted coil array. Such conducting traces may be fabricated along with the coil elements upon the thin-film layer in a similar fashion. Another terminal of the TCMB is connected to corresponding low-input-impedance preamplifier with a half-wavelength, 50-ohm-impedance coaxial cable, as shown in FIG. 8B.

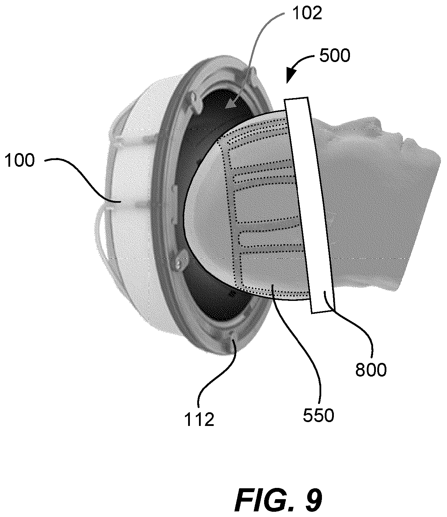

In some embodiments, the end ring may be completely sealed to the edge structures to form a watertight seal. In some embodiments, the end ring clamps down against the flexible material of the thin-film layers with enough pressure to prevent water or other liquids from passing the interface of the end ring and edge structures. In turn, the end ring is configured to engage and interface totally sealed with a tFUS transducer with a watertight seal. As such, water may be contained within a cavity formed between at least the outer thin-film layer, the tFUS transducer device, and the edge ring. FIG. 9 shows a customized coil array assembly, such as 200-A, with end ring 800 interfacing with tFUS transducer 100. In various embodiments, attachment mechanisms 112 of transducer 100 may be configured to engage and totally seal with end ring 800. Once fully sealed, cavity 102 within the transducer is fully sealed between the tFUS transducer and the coil array assembly. As such, a water bath or other liquid may be contained within cavity 102 without disrupting or interfering with the coil elements and other components of the coil array assembly.
The example customized thin-film coil array assembly 200-A (or 500) as described is wearable and fabricated to conform to the head of a particular patient. Coil array assembly 200-A provides the closest fit to minimize ultrasound attenuation, is best mechanically supported by the head, and satisfies MRI requirements of close proximity as well as multiple-channel parallel acceleration. It can also be reused by the same patient for subsequent or follow-up treatment. As an alternative embodiment, a thin-film coil array assembly may be fabricated to conform to one or more universally defined shapes in conjunction with a customized thin layer of gel pad lining.

### Universal Thin-Film Coil Array Assembly Configuration

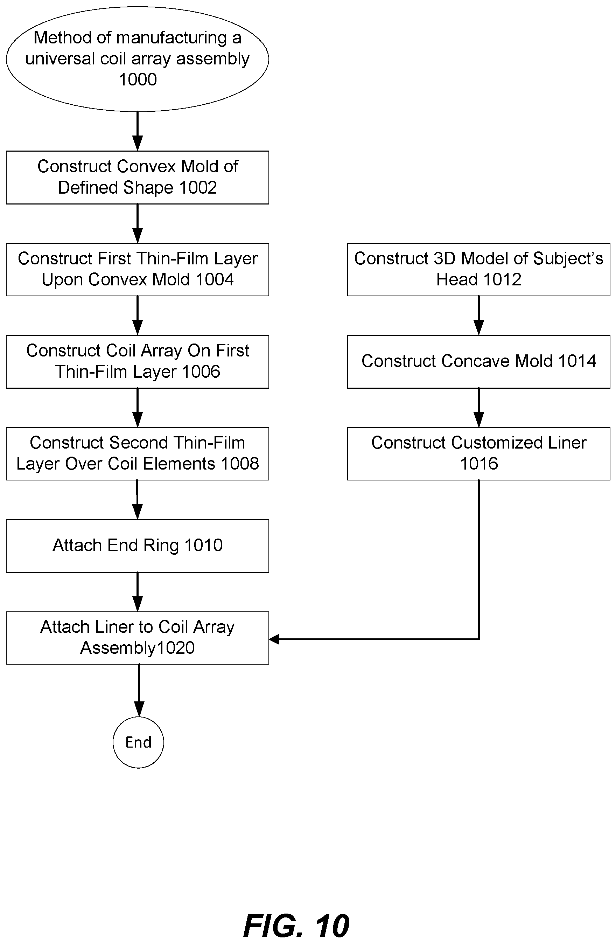

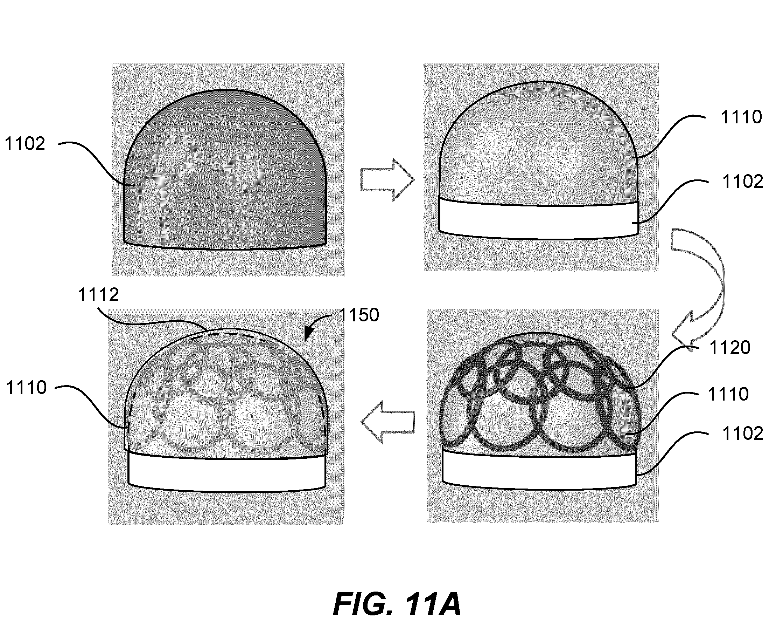

In another embodiment, instead of being customized to specific subject's head, the thin-film coil array assembly is fabricated over a fixed mold with a defined geometric shape of one or more sizes for the closest possible fit to the specific subject. This approach of a universal coil array assembly configuration is facilitated by a customized gel pad liner, fabricated to form a close fit to subject's head to ensure no void space is formed between the subject's head and the thin-film coil array cap thus avoiding ultrasound deflection or reflection. FIG. 10 shows an example process for constructing a universal coil array assembly, in accordance with one or more embodiments. FIG. 10 is described with reference to the following FIGS. 11A, 11B, 12, 13, and 14, which illustrate the configuration of the described coil array assemblies, and components, at various stages of construction.

At operation 1002, a convex mold of defined shape is constructed, such as convex mold 1102 shown in FIG. 11A. FIG. 11A shows an example convex mold 1102 and thin-film layers fabricated thereon, in accordance with one or more embodiments. Thus, the thin-film coil array assembly is fabricated to conform to the external surface of a fixed geometric shape of convex mold 1102. In various embodiments, convex mold may be constructed along with a concave counterpart (1202), further described below. The defined geometric shape of the convex mold may be of a size that is slightly larger than the size of an average human head, while small enough to fit inside a tFUS transducer device. In some cases, convex molds may be constructed in additional sizes to accommodate heads of different sizes. The convex mold may be constructed from various materials, including Polylactic Acid (PLA) and other material suitable for the various 3D printing techniques described herein.

At operation 1004, a first thin-film layer is constructed upon the convex mold. Similar to operation 304, first layer, or inner layer, of material is fabricated upon the convex mold to form first thin-film layer 1110. As described, such material may comprise TPU thin film. Next, a coil array is constructed upon the first thin-film layer at operation 1006. Coil array 1120 is shown positioned upon first thin-film layer 1110 in FIG. 11A.

As previously described with reference to operation 306, the coil array may comprise one or more conducting material layers for coil trace and one or more dielectric layers for distributed capacitors. In various embodiments, the coil pattern comprises a pattern of conductive traces and distributed capacitors configured into one or more coil elements. Coil array 1120 is shown as a configuration of overlapping rows of overlapping circular element loops, similar to that described in FIG. 2B. However, coil arrays of various patterns of conductive elements may be constructed at operation 1006, including, but not limited to the cylindrical configuration shown in FIG. 2A and FIG. 5C. Likewise, the customized thin-film coil array assembly constructed via method 300 may include a coil array of overlapping element loops as shown in FIG. 11A as an alternative to the cylindrical configuration shown in FIGS. 5A-5D.

As one example embodiment shown in FIG. 11A, coil array 1120 comprises up to 16 elements distributed over a hemi-spherical configuration positioned to cover or surround the top of the subject's head. There are two ascending rows of overlapping elements. However, the number of elements is not limited to 16. Additional rows of overlapping elements may also be implemented. The coil pattern of coil array 1120 may comprise three layers (similar to layers 610, 620, and 630 shown in FIGS. 6 and 7) of conducting traces-dielectric-conducting traces printed on top of the first thin-film layer one after the other. Similar to FIG. 7, adjacent coil loop elements may also be overlapped (positioned in different layers) to eliminate mutual inductance for coil array 1120, except the overlap may repeat itself in more directions than one for the example configuration.

At operation 1008, a second, or outer, thin-film layer is constructed over the coil elements, as well as the first thin-film layer to complete the thin-film coil array cap 1150. FIG. 11A shows second thin-film layer 1112 positioned over first thin-film layer 1110 (shown in dashed lines) in the bottom left diagram. As such, the coil array may be completely or mostly sealed between the first and second thin-film layers. As previously described, the first and second thin-film layers may be constructed by multi-dimensional printing equipment discussed earlier. As also previously explained air pockets or gaps between the thin-film layers and coil element are eliminated or reduced to the extent possible.

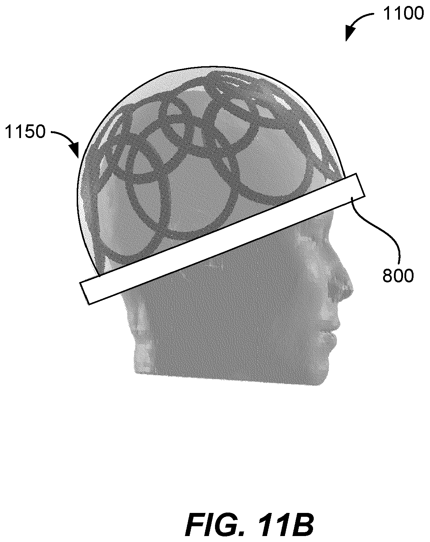

The completed thin-film coil array cap may then be removed from the convex mold and an end ring, such as end ring 800, is attached to the thin-film layers of the thin-film coil array cap at operation 1010, as previously discussed with reference to FIG. 8D. The end ring with tune/match boards housed inside is connected and sealed to the thin-film coil array cap 1150. FIG. 11B shows end ring 800 attached to the thin-film coil array cap 1150 forming coil array assembly 1100.

Coil array assembly 1100 may be universally used for any user with a head of appropriate size to fit within assembly 1100. However, because the universal coil array assembly is constructed to have a configuration corresponding to a mold 1102 of a predefined size and geometry, such assembly may not precisely fit any one particular subject or patient's head to allow for adequate elimination of any air gaps that may deflect the ultrasound beam or cause skin burn at the location of air gaps. Therefore, additional structures described herein may be constructed and implemented within such universal coil array assembly. For example, a personalized lining layer may be constructed and worn on patient's head, or otherwise placed on the inside of the thin-film coil array cap, before the assembly is put on top of patient's head to provide and maintain the closest possible fit without compromising the ultrasound transmitted by the transducers. The construction of personalized lining layers may be faster and much more economical than construction of a personalized thin-film coil array cap containing complex conductive traces. As such, creating a personalized lining layer allows the cost-effective use of a single universal thin-film coil array assembly by multiple patients along with respective personalized liners.

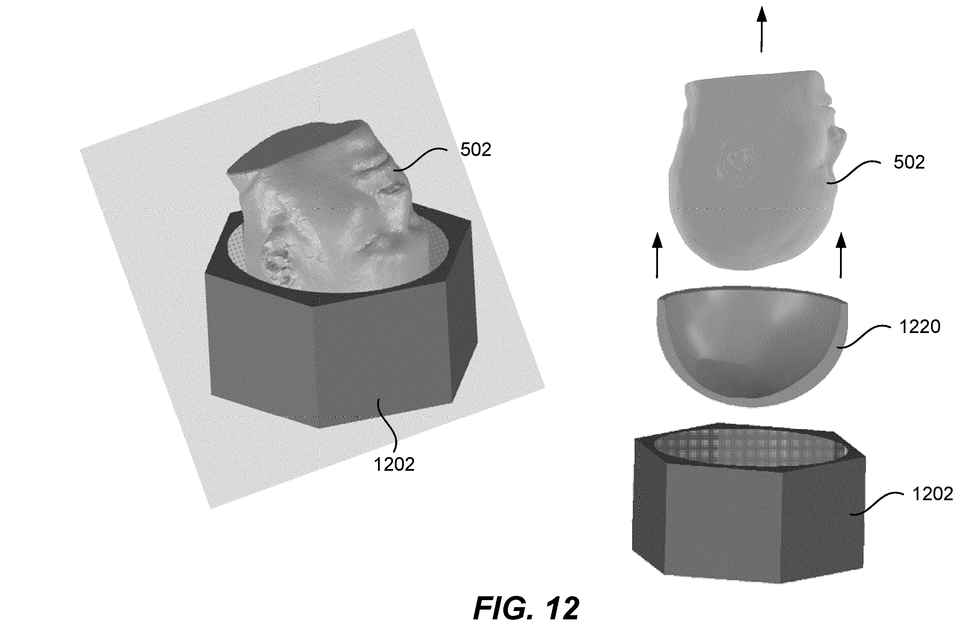

In some embodiments, an ultrasound transparent gel material is used to fill in the void space formed between the patient's head mold (502 shown in FIG. 5A) and the inner surface of the concave fixed-shape mold (1202 in FIG. 12), and allow to form a semi-rigid thin layer of personalized gel pad liner 1220 as in FIG. 12. Referring back to process 1000, additional operations are described for fabricating a patient specific lining layer to fit onto a particular patient's head and that functions as an effective interface between the patient's head and a thin-film cap portion of a thin-film coil array assembly. At operation 1012, a 3D model of the subject's head is constructed. As previously discussed with reference to operation 302, a physical model (such as a mold 502) may be constructed from 3D image data. However, only relevant portions of a 3D model may be used to create the physical mold to reduce material, cost, and fabrication time. At operation 1014, a concave mold is constructed. FIG. 12 shows an example concave mold 1202 for constructing personalized lining layers, in accordance with one or more embodiments. As shown in FIG. 12, concave mold 1202 may include a geometric interior surface shape that is identical to the predefined exterior shape of convex mold 1102. In other words, the external surface of convex mold 1102 may interface seamlessly with the interior surface of concave mold 1202.

In various embodiments, concave mold 1202 and the patient-specific head mold 502 are used together to form a customized liner at operation 1016. As the concave mold 1202 is a counterpart to convex mold 1102, the inner surface of mold 1202 should correspond exactly to the surface of convex mold 1102. When the patient-specific head mold is placed inside of the fixed-shape concave mold, a void space is formed. In some embodiments, head mold 502 may be positioned within the concave mold at a particular distance to maintain a minimum distance from the surface of the concave mold at every point along head mold 502. In some embodiments, head mold 502 may be positioned to within the concave mold to minimize the distance between all points of head mold 502 from the surface of the concave mold, while maintaining the minimum distance requirement. In some implementation, a landmark on the head mold may be devised to align the molds.

The customized liner may be formed by filling the void space with an ultrasound transparent material that will cure into a flexible or semi-rigid structure. For example, a selected ultrasound gel material may be filled into the void space and allowed to form into a semi-rigid thin gel pad, or customized liner 1220. In various embodiments, the material may be cured at room temperature or with heat. Once the customized liner has formed, head mold 502 may be removed to allow access to remove the customized liner, as shown in FIG. 12. Once formed, the customized liner should include an exterior surface corresponding to the inner surface of the mold 1202, and an interior surface corresponding to the surface of the head mold. Some additional machining may be performed to shape or finish the customized liner.

Another way the customized liner can be fabricated is by constructing the liner layer directly onto the head mold. For example, the head mold may be coated with liner material such that the interior surface of the liner conforms to the shape of the head mold. Then excess liner material may be shaped or machined such that the exterior surface conforms to the shape of the universal thin-film coil array cap. As another example, liner material may be 3D or higher dimensionally printed directly onto the head mold to have the required exterior geometric shape. In some embodiments, in order to provide more structural support for the liner layer during manufacturing, additional thin-film layers may be implemented. For example, an inner thin-film layer, such as TPU, may first be constructed onto the head mold much like the first thin-film layer. Then, the liner material may be constructed over the inner thin-film layer as discussed above. In certain embodiments, an additional outer thin-film layer may be constructed over the formed liner layer to seal the liner layer within the inner and outer thin-film layers.

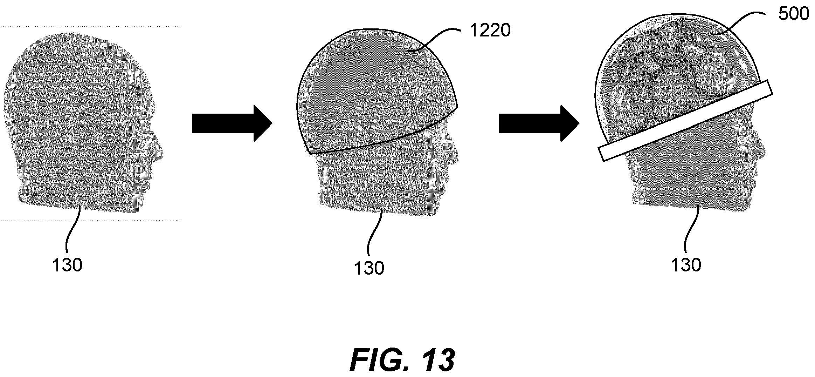

At operation 1020, the customized liner is attached to the coil array assembly. FIG. 13 illustrates an example flow process for placement of a universal thin-film coil array assembly onto a patient's head, in accordance with one or more embodiments. In some embodiments, the customized liner is first placed or worn onto the patient's head 130, as shown in FIG. 13. The universal thin-film coil array assembly 1100 may then be placed onto the external surface of the liner with complementary geometric profile. However, in some embodiments, the customized liner may first be interfaced with the interior surface of the first (inner) thin-film layer of the coil array assembly 1100 prior to placement on the subject's head. In some embodiments, the customized liner may be secured and/or sealed along with the assembly by the end ring. Thus, a universal thin-film coil array assembly can be used for multiple patients, each having their own customized liner as a consumable.

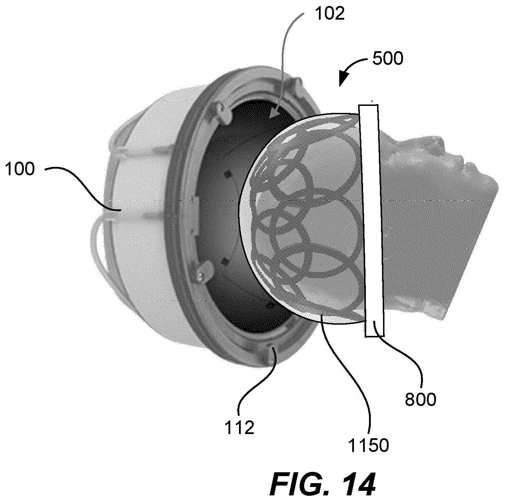

With reference to FIG. 14, shown is an interface between a universal thin-film coil array assembly 1100 and the frame of a tFUS transducer device 100, in accordance with one or more embodiments. In some embodiments, assembly 1100 may alternatively interface with a tFUS transducer which has a transducer array distributed in a ring shape structure (as described with respect to transducer 100-B in FIG. 2B) in order to minimize interference with the particular configuration of coil elements of assembly 1100. In various embodiments, attachment mechanisms 112 of transducer 100 may be configured to completely seal onto the end ring 800. Once fully sealed, cavity 102 within the transducer is fully sealed between the tFUS transducer and the coil array assembly. As such, a water bath or other liquid may be contained within cavity 102 without disrupting or interfering with the coil elements and other components of the coil array assembly.

### Conclusion

This disclosure is not limited by the design of the coil array, number of coil elements or loops, the shape or trace of the loops, or its spatial distribution. The 8-channel array shown in FIG. 8C and the 16-channel array in FIG. 11B are only examples of possible configurations. As one of the major considerations in the tight integration of the head coil array and the tFUS transducer device is the mutual interference between the two modalities, one simple principle is to maintain the orthogonality of the two fields, ultrasound field in the direction of the beam and the magnetic field normal to the coil loop. Since the conventional tFUS transducer array consists of hundreds of transducer elements which are typically distributed over a hemispherical shell, an ideal placement of the coil loops would arrange the loops along the side of a cylinder as shown in the configuration in FIG. 2A as well as the example given in FIG. 5C.

Different examples of the apparatus(es) and method(s) disclosed herein include a variety of components, features, and functionalities. It should be understood that the various examples of the apparatus(es) and method(s) disclosed herein may include any of the components, features, and functionalities of any of the other examples of the apparatus(es) and method(s) disclosed herein in any combination, and all possible variations are intended to be within the spirit and scope of the present disclosure. Many modifications of examples set forth herein will come to mind to one skilled in the art to which the present disclosure pertains having the benefit of the teachings presented in the foregoing descriptions and the associated drawings.

Therefore, it is to be understood that the present disclosure is not to be limited to the specific examples illustrated and that modifications and other examples are intended to be included within the scope of the appended claims. Moreover, although the foregoing description and the associated drawings describe examples of the present disclosure in the context of certain illustrative combinations of elements and/or functions, it should be appreciated that different combinations of elements and/or functions may be provided by alternative implementations without departing from the scope of the appended claims. Accordingly, parenthetical reference numerals in the appended claims are presented for illustrative purposes only and are not intended to limit the scope of the claimed subject matter to the specific examples provided in the present disclosure.

## Claims

1. A magnetic resonance imaging (MRI) receiver coil device comprising:
a thin-film substrate layer configured in a dome shape;
a coil array positioned around a circumference of an exterior surface of the thin-film substrate layer;
a thin-film cover layer positioned over the exterior surface of the thin-film substrate layer such that the coil array is positioned between the thin-film cover layer and the thin-film substrate layer; and
an end ring engaging the thin-film substrate layer and the thin-film cover layer such that a watertight seal is formed around the coil array between the thin-film substrate layer, the thin-film cover layer, and the end ring.

2. The MRI receiver coil device of claim 1, wherein the dome shape conforms to a geometric profile of a head of a particular subject, or a predetermined geometric profile, or wherein the MRI receiver coil device further comprises a lining layer configured to conform to a geometric profile of a head of a particular subject.

3. The MRI receiver coil device of claim 1, wherein the thin-film substrate layer or the thin-film cover layer comprise thermoplastic polyurethane.

4. The MRI receiver coil device of claim 1, wherein the thin-film substrate layer having a thickness of no greater than 0.1 millimeters.

5. The MRI receiver coil device of claim 1, wherein the coil array comprises a plurality of coil elements, each coil element comprising a loop of conductive trace material with at least one capacitive segment.

6. The MRI receiver coil device of claim 5, wherein adjacent coil elements overlap at overlapping segments, wherein the coil array further comprises dielectric traces positioned within the overlapping segments to electrically isolate the adjacent coil elements.

7. The MRI receiver coil device of claim 6, wherein the coil array comprises:
a first layer of conductive material on the exterior surface of the thin-film substrate layer, the first layer of conductive material having a first pattern;
a second layer of conductive material on the exterior surface of the thin-film substrate over the first layer of conductive material, the second layer of conductive material having a second pattern, wherein the first pattern and the second pattern overlap at the overlapping segments and the capacitive segments; and
a layer of dielectric material positioned between the first layer of conductive material and the second layer of conductive material at the overlapping segments and the capacitive segments, wherein a portion of the overlapping segments form one or more capacitors.

8. The MRI receiver coil device of claim 6, wherein the conductive material comprises a conductive ink, the conductive ink comprising one or more of the following: gold, copper, silver, graphene, and metal flakes.

9. A method of constructing a magnetic resonance imaging (MRI) receiver coil device, the method comprising:
fabricating a thin-film substrate layer over a mold having a dome shape;
fabricating a coil array around a circumference of an exterior surface of the thin-film substrate layer;
fabricating a thin-film cover layer positioned over the exterior surface of the thin-film substrate layer such that the coil array is positioned between the thin-film cover layer and the thin-film substrate layer; and
engaging an end ring to the thin-film substrate layer and the thin-film cover layer to form a watertight seal around the coil array between the thin-film substrate layer, the thin-film cover layer, and the end ring.

10. The method of claim 9, further comprising forming the dome shape to conform to a geometric profile of a head of a particular patient, or to a predetermined geometric profile, or further comprising configuring a lining layer of the MRI receiver coil device to conform to a geometric profile of a head of a particular patient.

11. The method of claim 9, wherein the thin-film substrate layer or the thin-film cover layer are fabricated as a thermoplastic polyurethane layer, and wherein the fabricating the thin-film substrate layer comprises fabricating the thin-film substrate layer to have a thickness of no greater than 0.1 millimeters.

12. The method of claim 9, wherein the fabricating the coil array further comprises fabricating a plurality of coil elements, each coil element comprising a loop of conductive trace material with at least one capacitive segment.

13. The method of claim 12, wherein fabricating the coil array includes overlapping adjacent coil elements at overlapping segments, and positioning dielectric traces within the overlapping segments to electrically isolate the adjacent coil elements.

14. The method of claim 13, wherein fabricating the coil array comprises:
fabricating a first layer of conductive material on the exterior surface of the thin-film substrate layer, the first layer of conductive material having a first pattern;
fabricating a second layer of conductive material on the exterior surface of the thin-film substrate over the first layer of conductive material, the second layer of conductive material having a second pattern, wherein the first pattern and the second pattern overlap at the overlapping segments and the capacitive segments; and
fabricating a layer of dielectric material between the first layer of conductive material and the second layer of conductive material at the overlapping segments and the capacitive segments, wherein a portion of the overlapping segments form one or more capacitors.

15. A system comprising:
a transcranial focused ultrasound (tFUS) transducer device comprises multiple transducer elements for transmitting multiple ultrasound beams;
a magnetic resonance imaging (MRI) receiver coil device comprising:
a thin-film substrate layer with a coil array disposed on an exterior surface of the thin-film substrate layer and covered by a thin-film cover layer, and
an end ring engaging the thin-film substrate layer and the thin-film cover layer such that a watertight seal is formed around the coil array between the thin-film substrate layer, the thin-film cover layer, and the end ring; and wherein the coil array is electrically connected to send received signals to MRI receivers through tune and match circuit boards (TMCBs) enclosed in the end ring;
wherein the MRI receiver coil device is configured to be worn onto the subject's head with a close and airtight fit;
wherein the transcranial focused ultrasound (tFUS) transducer device is configured to
seal over and onto the MRI receiver coil device therein with a watertight fit; and wherein the system is configured to simultaneously operate the MRI receiver coil device
with the tFUS transducer device to provide a real-time image navigation, including targeting, positioning, aiming, real-time dose monitoring and controlling operations of a tFUS treatment procedure.
